# EUROPEAN PATENT APPLICATION

(11) **EP 2 787 084 A1**
(43) Date of publication of application: **08.10.2014**
(21) Application number: 13305443.7
(22) Date of filing: 05.04.2013
(51) Int. Cl.: C12Q 1/68

(54) **Biomarkers for the identification of chemosensitivity**

(71) Applicant: Les Laboratoires Servier, 92284 Suresnes Cedex (FR)
(72) Inventor: Birnbaum, Daniel, 13720 La Bouilladisse (FR); Charafe-Jauffret, Emmanuelle, 13012 Marseille (FR); Ginestier, Christophe, 13009 Marseille (FR); Salvador, Marion, 13009 Marseille (FR)

(57) **Abstract**

The instant application provides biomarkers for the identification of chemosensitivity in patient having cancer. In a particular embodiment the invention provides XIST for the identification of chemosensitivity in breast cancer patient, wherein a reduced expression of XIST in the breast cancer cells of the patient indicates that the breast cancer stem cells in the patient may be successfully treated with HDAC inhibitor.

## Description

The present disclosure relates to methods for predicting chemotherapy sensitivity. In certain embodiments, the invention relates to the field of methods for predicting HDAC (histone deacetylase) inhibitor sensitivity, such as abexinostat sensitivity, in a CSC (cancer stem cell) population of breast cancer by detecting a low XIST (X inactive specific transcript) expression.

The most significant hurdle to developing new and efficacious cancer drugs is the absence of proven strategies that enable responsive tumors to be identified and treated with the most effective drug. A major goal of oncology is therefore to identify biomarkers that predict the response of tumors before treatment is started. A companion biomarker would enable patients to be stratified and also enable prediction of chemotherapy response. HDAC inhibitors have been proposed as an alternate therapy to conventional therapeutics in solid malignancies. Nevertheless, these compounds are only efficient at high concentrations. Thus, the effect of HDAC inhibitors might at least in part be the results of non-specific side effects rather than the consequences of inhibiting HDAC *per se.* Furthermore, there is no existing biomarker able to predict HDAC inhibitor efficiency.
There is a need in the art for a useful approach to assess a patient's chemosensitivity and to provide one or more markers for evaluating said chemosensitivity.

The present invention provides a method of evaluating chemotherapy sensitivity in a patient in need thereof. The method of evaluating chemotherapy sensitivity comprises the assessment of the number of X chromosome in a sample from the patient and the comparison of the number of X chromosome in the sample with the number of X chromosome reference from a control sample. This method is **characterized in that** a number of X chromosome that is identical or inferior to the reference is indicative of chemotherapeutic sensitivity in the patient.

The present invention provides a method of evaluating chemotherapy sensitivity in a patient in need thereof. The method of evaluating chemotherapy sensitivity comprises the assessment of the level of expression of XIST gene in a sample from a patient and the comparison of the level of expression of XIST gene in the sample with the level of expression of reference XIST gene from a control sample. This method is **characterized in that** a level of expression of XIST gene that is inferior to the reference is indicative of a chemotherapeutic sensitivity in the patient.

"XIST" is referred to a XIST gene (ENSG00000229807) or the encoded XIST RNA regardless of the origin of the sequence, i.e. human, mouse... XIST or inactive specific transcript is an RNA gene on the X chromosome that acts as major effector of the X inactivation process.

Therefore, the present invention provides the number of X chromosome and the level of expression of XIST gene as markers or biomarkers for chemosensitivity in a patient. The terms "marker" or "biomarker" are used interchangeably herein and refer to the expression or level of a gene, a group of genes correlated with a certain condition wherein the gene or the group of genes is a marker for that condition.

In some embodiments the invention provides methods wherein the number of X chromosome reference is the X chromosome monosomy. By "monosomy" is meant a form with the presence of only one chromosome.
In others embodiments the invention provides methods wherein the number of X chromosome reference is the X chromosome disomy. By "disomy" is meant a form with two replica copies of a single homolog of a chromosome.

In certain embodiments the present invention relates to methods wherein the level expression of reference XIST gene is the level expression of XIST gene in a disomic sample.

In predicting chemotherapy sensitivity of a tumor, cut-off levels are different according to the biomarkers. The cut-off level to determine low XIST expression for example in breast cancer tissue is defined as below the level expression of XIST gene in a disomic sample. This threshold corresponds to the expression of XIST gene on Xi (inactivated X chromosome). Therefore the low XIST expression corresponds to an absence of expression of XIST on Xa (active X chromosome).
The cut-off level of the number of X chromosome is accurate. Actually, the cut-off level of the number of X chromosome to determine the patient sensitive to HDAC inhibitor corresponds to one or two copies of X chromosome. For example breast cancer tissue exhibiting more than two copies of X chromosome will be identified as resistant to HDAC inhibitor chemotherapy.

In other embodiments, if the marker of the invention, i.e. the number of X chromosome is higher than the number of X chromosome reference is indicative of a patient's potential resistance to chemotherapy, thus the patient will not be selected for chemotherapy treatment. If the marker of the invention is the level of expression of XIST gene which is identical or higher than the reference level of expression of XIST gene then this level of the marker is indicative of a patient's potential resistance to chemotherapy. The X chromosome polysomy in a human patient is correlated with a lack of chemosensitivity to certain drug and preferably a lack of sensitivity to HDAC inhibitor. The "polysomy" is a condition in which a diploid organism has at least one more chromosome than normal, there may be three or more copies of the chromosome.

By "chemotherapy sensitivity" or "chemosensitivity" is meant that the chemotherapeutic agent or other compound has an effect on the cancer cell.
Cancers for which the prognostic of the present invention provide results for chemosensitivity include cancers such as breast cancer, ovarian cancer, cervical cancer, lymphoma and hematology diseases.

Resistance to conventional therapeutic agents in cancer may be sustained by a fraction of cancer cells within the tumor, the cancer stem cells, which are able to self-renew and differentiate, giving rise to the bulk of the tumor. The present invention provides methods for identifying a cancer patient who would benefit from a certain chemotherapeutic treatment wherein the patient has cancer related to cancer stem cells. In breast cancer in particular, this population has been shown to resist to conventional chemotherapy and radiation, suggesting that it will be imperative to target all CSC subsets within the tumor to prevent relapse and metastasis. In a particular embodiment, the methods of the invention relates to patient having a cancer related to cancer stem cells and specifically patient having breast cancer stem cells.

In some embodiments, the chemosensitive patients identify thanks to the marker XIST or the number of X chromosome are sensitive to histone deacetylase inhibitor. HDAC inhibitor is a class of compounds that interfere with the function of histone deacetylase, i.e. enzymes regulating the acetylation level of chromatin, together with a variety of non-histone proteins.
The invention provides methods whereby measurements of low expression of XIST gene or X chromosome monosomy or disomy in one or more cancer cells of a patient identify these cancer cells as cells being sensitive to treatment by one or more HDAC inhibitor such as abexinostat, vorinostat, belinostat, panobinostat, entinostat, mocetinostat, resminostat, givinostat, quisinostat, romidepsin, valproic acid.

In certain embodiments the present invention relates to methods encompassing the detection of the level of expression of XIST gene or the number of X chromosome in a tumor sample from the patient. Preferably, the patient tumor sample is a breast tumor sample.

The level of expression of XIST gene serves as a marker for chemosensitivity in a patient as described in the present invention. There are many methods known in the art for determining the level of expression of XIST gene in a given sample. The level of expression of XIST gene is measured by determination of its level of transcription using DNA array, quantitative RT-PCR (reverse transcription polymerase chain reaction) or RNA FISH (Fluoresence In Situ Hybridization).
A nucleic acid array refers to nucleic acid members attached to a support. In certain embodiment the nucleic acid member attached to the surface of the support is DNA, either cDNA or oligonucleotides corresponding to the biomarker XIST and the total cellular RNA is applied to the array. The present invention relates to an array comprising a nucleic acid which binds to the biomarker XIST for the determination of chemosensitivity to a HDAC inhibitor such as abexinostat.
In addition to qualitative study of RNA expression levels, RT-PCR is utilized for quantification of RNA by incorporating qPCR (real time polymerase chain reaction) into the technique. The combined technique qRT-PCR is used to determine the mRNA transcript of XIST as biomarker for breast cancer stem cells. To perform cyclic polymerase mediated amplification reaction according to the invention, the primers (XIST: cat # Hs01077163_m1) should enable a specific hybridization or annealing to opposite strands of the target XIST DNA.

RNA-FISH is a technique derived from classical FISH allowing the detection of localizes specific RNA targets (mRNA, IncRNA and miRNA) in cells. The present invention relates to RNA-FISH using fluorescent probes, such as probe used in the following publication: "X inactive-specific transcript RNA coating and genetic instability of the X chromosome in BRCA1 breast tumors Vincent-Salomon A, Ganem-Elbaz C, Manié E, Raynal V, Sastre-Garau X, Stoppa-Lyonnet D, Stern MH, Heard E. Cancer Res. 2007 Jun 1;67(11):5134-40"; that bind to only Xist LncRNA. Fluorescence microscopy is used to find out where the fluorescent probe is bound for the detection of XIST-expressing cells.

The present invention relates to method encompassing the detection of the number of X chromosome in one or more tumor cells of a human patient which is measured by SKY (spectral karyotyping), M-FISH (Multiplex Fluorescence *in situ* Hybridization), array CGH (Array-comparative genomic hybridization) or FISH on paraffin section.
SKY and M-FISH are molecular cytogenetic techniques used to simultaneously visualize all the pairs of chromosomes in an organism. Fluorescently labeled probes for each chromosome are made by labeling chromosome-specific DNA with different fluorophores. Consequently each chromosome can be specifically identified. These techniques are used to evaluate the number of X chromosome for the determination of chemosensitivity to HDAC inhibitors.
Alternatively, array CGH is a technique to detect genomic copy number variations at high resolution level. DNA from a test sample and normal reference sample are labeled differentially, using different fluorophores, and hybridized to several thousand probes. The probes are derived from most of the known genes and non-coding regions of the genome, printed on a glass slide. The fluorescence intensity of the test and of the reference DNA is then measured, to calculate the ratio between them and subsequently the copy number changes for a particular location in the genome. This technique is used to create a virtual karyotype used to determine the number of X chromosome as a biomarker for HDAC inhibitor treatment response.
All these techniques need either metaphase spread or extracted DNA. Detection of the number of X chromosome can also be determined by FISH (Fluoresence In Situ Hybridization) on paraffin section. The present invention relates to FISH on cancer samples embedded in paraffin using fluorescent probes that bind to one region of the X chromosome. Fluorescence microscopy is used to find out where the fluorescent probe is bound to count X chromosome.

The present invention provides a kit comprising at least one pair of primers specific for XIST gene that is a marker for chemosensitivity in a patient that has a cancer, at least one reagent for amplification of the XIST gene and instructions for use. Preferably, the kit is a quantitative RT-PCR kit.

The present invention also relates to a kit comprising at least one probe specific for the number of X chromosome or specific for XIST gene that both are markers for chemosensitivity in a patient that has a cancer and instructions for use. Preferably, the kit is a FISH on paraffin section kit or a RNA-FISH kit.

In the kit of the present invention, the markers for chemosensitivity are markers for sensitivity to a HDAC inhibitor, such as abexinostat. Furthermore, the use of the kit according to the present invention is preceded by an RNA extraction from the tested sample, such as breast tumor sample.

The invention relates to the use of XIST gene expression or number of X chromosome for assessing a patient sample in vitro for susceptibility to treatment with an HDAC inhibitor such as abexinostat.

The present invention concerns an HDAC inhibitor for use in treating a cancer in a patient who is identified with one of the methods described previously.
In other embodiments the invention relates to abexinostat for use in treating a breast cancer related to cancer stem cells in a patient who is identified with one of the methods described previously.

The present invention is illustrated by the following Figures and Examples, without being limited thereby :
Figure 1 : BCLs (breast cancer cell lines) display two drug-response profiles to abexinostat
Figure 2 : HDACi (HDAC inhibitor) treatments define two-drug response profiles in BCLs
   A : response profiles to SAHA (vorinostat)
   B: response profiles to valproic acid
   C : for all three HDACi compounds tested, BCLs conserved their high- or low-dose sensitive response profile
Figure 3 : Conventional molecular parameters do not predict drug-response to abexinostat
   A : classification of BCLs according to their increasing IC50s
   B : molecular subtypes L=luminal, B=basal, M=mesenchymal ER=estrogen PR=progesterone
   Other parameters black box=positive, white box=negative, oblique feature=non-determined
Figure 4 : HDAC activity is impaired by treatment independently of drug-response profiles
   A : basal HDAC activity does not predict BCLs response to abexinostat
   B : abexinostat inhibits HDAC activity in both high- and low dose sensitive BCLs
Figure 5 : long non-coding RNA XIST predicts response to abexinostat
   A : XIST is the most differentially expressed gene between the two BCL populations
   B : Low-dose sensitive BCLs present an X monosomy whereas high-dose sensitive BCLs are disomy-or polysomic for X chromosome
Figure 6 : Abexinostat targets the CSC population in Patient-Derived Xenografts (PDX) with low XIST expression.
   A : Tumor growth kinetic of PDX [CRCM226X (XIST^{low}), CRCM392X (XIST^{med})] treated with docetaxel, abexinostat or placebo (arrow indicates beginning of treatment) Error bars represent mean ± SD.
   B : The ALDEFLUOR-positive population of PDXs CRCM226X (XIST^{low}), CRCM392X (XIST^{med}) and CRCM389X (XIST^{high}) after 3 weeks of treatment by abexinostat or docetaxel
   C : 3-weeks treated PDXs (right: CRCM226X, middle: CRCM392X, left: CRCM389) were reimplanted into new mice and tumor growth was monitored. Error bars represent mean ± SD.

### Example 1 : HDAC inhibitor treatment defines two drug-response profiles in breast cancer cell lines

A large series of cell lines (16 BCLs) were exposed to increasing concentrations (150nM - 2.5µM) of the abexinostat, for 72 hours. These series represent all the molecular diversity observed in breast cancers.

**Cell lines.** A total of 16 breast cancer cell lines (BCLs) were used for the study. HCC1500, HCC1937, HCC1954, Hs 578T, MCF7, MDA-MB-134, MDA-MB-231, MDA-MB-361, MDA-MB-436, SK-BR-7, T-74D and ZR-75-30 were obtained from American type culture collection (http://www.atcc.org/). SUM149 and SUM159 were obtained from Dr S. Ethier's (Karmanos Cancer Center, Detroit, MI, USA). S68 was obtained from Dr V. Castros (Université de Rennes, France) and Br-Ca-MZ-01 from Dr V. Möbus (University of Ulm, Germany). All BCLs tested were derived from carcinomas. BCLs were grown using the recommended culture conditions (Neve et al, Cancer Cell 2006).

**Drugs.** BCLs were continuously treated for 72h in adherent conditions with Histone Deacetylase Inhibitors (HDACi): abexinostat, SAHA and valproic acid. For experiments abexinostat was prepared in a 23,1 mM stock solution, SAHA in a 0.5M stock solution, in Dimethyl Sulfoxide (DMSO) and stored at -20°C. Valproic acid was prepared in a 1M stock solution in Phosphate Buffered Saline (PBS) and stored at 4°C. For experiments, cells were treated with respective IC50s. DMSO or PBS were used as vehicle control (C<0.1%).

**Cell viability.** Inhibitory Concentrations 50 (IC50s) were evaluated using 3-(4,5-dimethylthiazol-2-yl)-5-(3-carboxymethoxyphenyl)-2-(4-sulfophenyl)-2H-tetrazolium (MTS) assay. BCLs were plated in adherent condition in 96-well plates at 5,000 cells per well; except for SUM159 plated at 1,000 cells per well. After 24h, treatment with serial dilutions of HDACi was started. The effect of treatment on cell viability was estimated after 72h by addition of 100 µL MTS solution (5 mg/mL in PBS) in each well. Cells were then incubated for 1 h at 37°C. Absorbance was measured at 540 nM in a fluorescence plate reader.

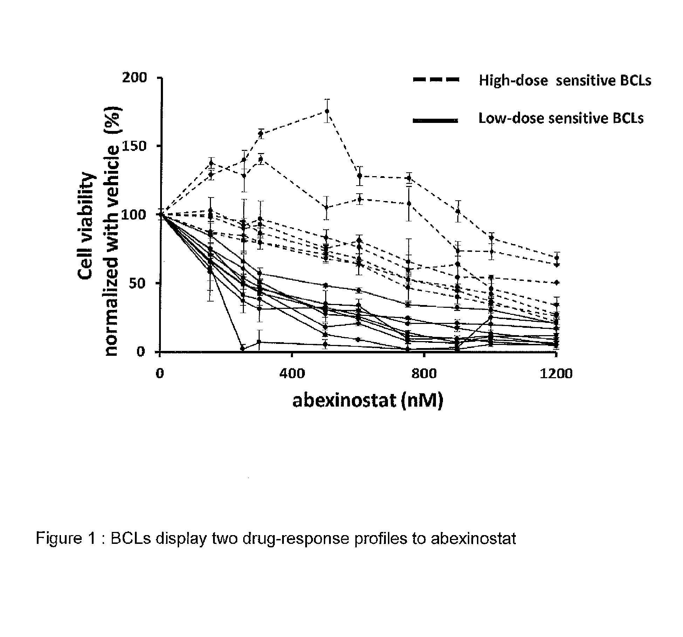

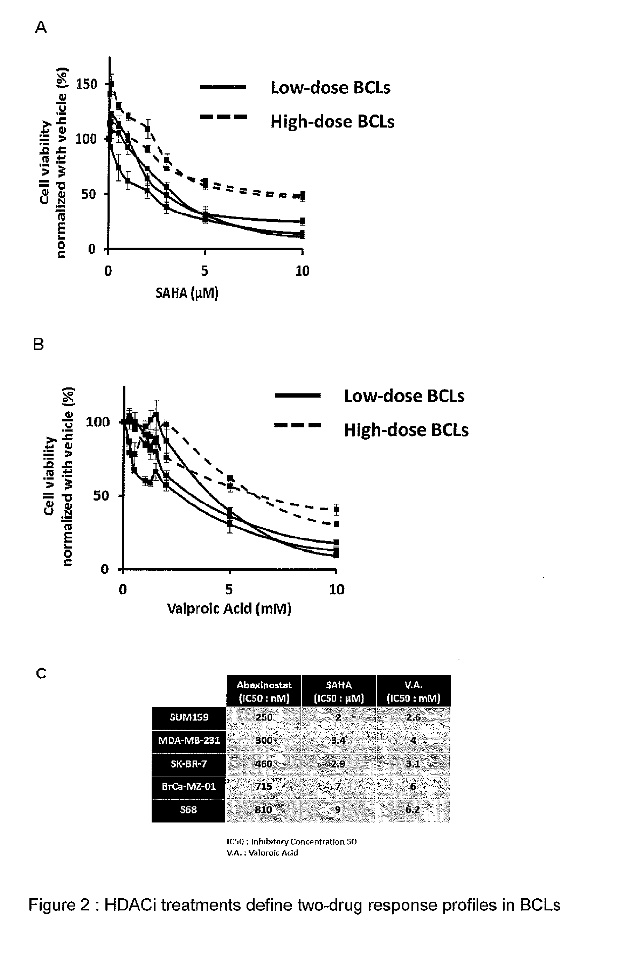

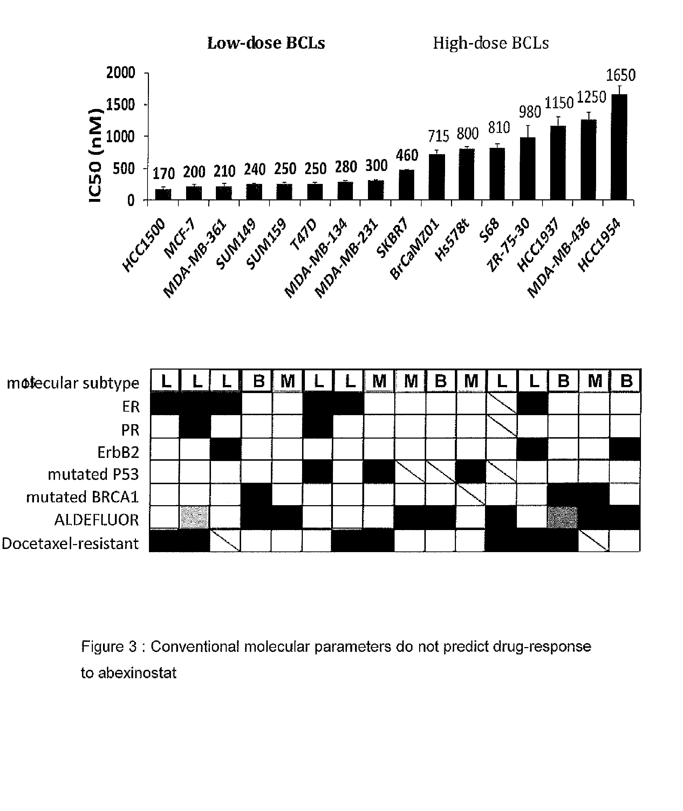

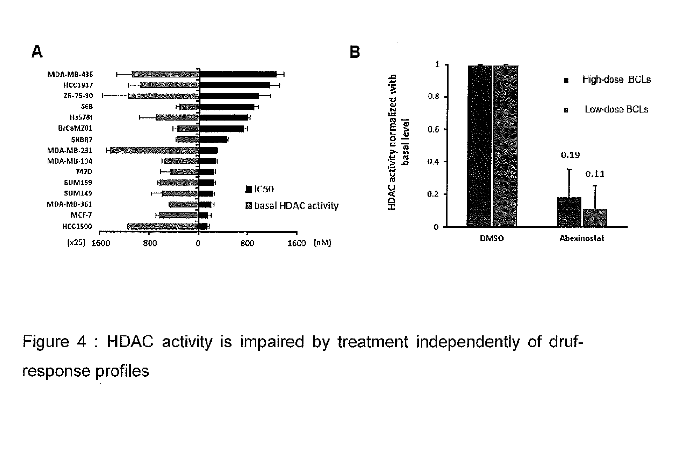

Nine BCLs were significantly sensitive to low-dose of abexinostat with an IC50 comprised between 170nM and 460nM whereas seven BCLs sensitive to high-dose of abexinostat presented an IC50 superior to 700nM (IC50s range: 715nM - 1650 nM) (Figure 1). Both drug-response profiles to abexinostat were confirmed using other HDACi compounds (SAHA and Valproic Acid) (Figure 2). To explain the differential drug-response of BCLs to HDACi treatment BCLs have been grouped according to their molecular features. None of the molecular parameters tested could predict drug-response to HDACi (Figure 3). To exclude indirect cytotoxic effect of HDACi treatment, we compared our abexinostat drug-response profile to the one of docetaxel, a conventional chemotherapeutic agent used to treat breast cancer. Both drug-response profiles were totally distinct, reinforcing HDACi treatment effect specificity (Figure 3). Histone deacetylase activity is measured before and after abexinostat treatment in the BCL series (Figure 4A, 4B). Intrinsic histone deacetylase activity of BCLs was not correlated to BCL's drug-response profile and both low-dose sensitive and high-dose sensitive BCLs presented a similar extinction of HDAC activity after treatment. Altogether these results indicate that abexinostat treatment inhibits specifically HDAC activity in all BCLs tested, independently of their drug-response profile.

### Example 2 : Identification of XIST and number of X chromosome as biomarkers

A biomarker is needed to predict drug-response to abexinostat of patients with breast cancer. None of the classical molecular parameters tested could predict drug-response of BCLs to abexinostat (Figure 3). Thus, a gene expression analysis and a comparison of transcriptional profiles of low-dose and high-dose sensitive BCLs have been performed.

**Gene expression profiling** RNA expression data was collected from the study Charafe-Jauffret et al;, Oncogene, 2006 done with Affymetrix U133 Plus 2.0 human oligonucleotide microarrays. A supervised analysis is applied based on volcano plot analysis, where fold-change and statistical difference between groups were evaluated for each probe set. Probabilities were computed using linear models with empirical Bayes statistic included in the limma R package.

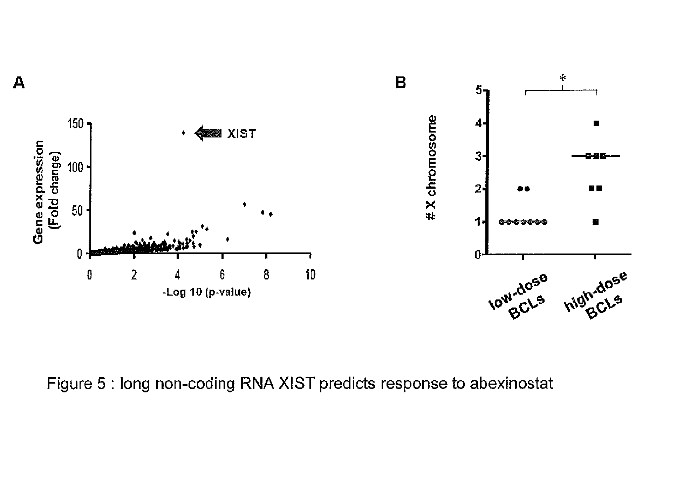

An overexpression of XIST IncRNA up to 139-fold (T test pval < 0.00001; FDR qval: 0.03) is identified in high-dose sensitive BCLs compared to low-dose sensitive BCLs (Figure 5A).
Several studies have reported genomic instability of X chromosomes (loss of Xi, duplications of Xa) and dysregulation of XIST in breast, ovarian, cervical, prostate cancers, testicular germ cell tumors and lymphoma (Agrelo, EMBO molecular medicine 2009; Weakley, World J Surg 2011). Furthermore, a strong correlation between X chromosomes number and drug-response to abexinostat is observed. Low-dose sensitive BCLs presented essentially X chromosome monosomy whereas high-dose sensitive BCLs presented X chromosome normo- or polysomy (p<0.007) (Figure 5B). All together these results suggest that XIST IncRNA expression and/or number of X chromosomes may be used as a biomarker predicting abexinostat treatment response.

### Example 3 : HDAC inhibitor treatment reduces the breast cancer in patient-derived xenografts (PDXs) with low XIST expression

To confirm the impact of abexinostat treatment on the CSC population from breast cancers with low XIST expression, three different patient-derived xenografts (PDXs) with distinct Xist expression level (CRCM226X, Xist^{low}; CRCM392X, Xist^{med}; CRCM389X, Xist^{high}) were utilized.

**Animal models** Cells from these PDXs were transplanted orthotopically into fat pads of NOD/SCID mice without cultivation in vitro. Single cancer cells (1,000,000 cells CRCM226X and CRCM389X, 125,000 cells CRCM392X) are injected into fat pads of NOD/SCID mice and tumor growth are monitored. When the tumor size was approximately 150 mm³, treatment is started with abexinostat (i.p, 12.5 mg/Kg, twice a day, 5 out of 7 days for three weeks) or docetaxel (10 mg/kg weekly for three weeks) or placebo.
Six mice were injected for each PDX and for each group. After 1, 2 and 3 weeks of treatment, two mice from each group were sacrificed according to ethic statements. Tumors were dissociated and cells were analyzed for the ALDEFLUOR phenotype and tumorsphere formation capacity. Cells from 3-week treated mice were reimplanted into secondary NODISCID mice with injection of 1,000 cells for each treated tumor.

**ALDEFLUOR assay** The ALDEFLUOR kit (Stem Cell Technologies) was used to isolate the population with high aldehyde dehydrogenase (ALDH) enzymatic activity using an LSR2 cytometer as previously described (Ginestier et al, Cell Stem Cell, 2007). For BCLs cultivated in adherent condition, ALDEFLUOR-positive populations were evaluated after 72-hour treatment with HDACi (IC50s) or vehicle. Primary human xenografts CRCM226X, CRCM389X and CRCM392X were harvested on the 3rd week of treatment with abexinostat, docetaxel or placebo, dissociated and stained for ALDEFLUOR analysis. To eliminate cells of mouse origin from the xenotransplanted tumors, staining with an anti-H₂K^{d} biotin antibody (1/200; BD Biosciences) followed by staining with a secondary antibody labeled with streptavidin e450 (1/200; eBioscience) was used. **Tumorsphere assay** BCLs were grown in adherent conditions under abexinostat treatment (IC50s) or vehicle for 72h, then seeded as single cells in ultra-low attachment plates at low density (1,000 viable cells per ml). Tumorspheres were grown in a serum-free mammary epithelium basal medium. The capacity of cells to form primary tumorspheres was quantified under microscope.

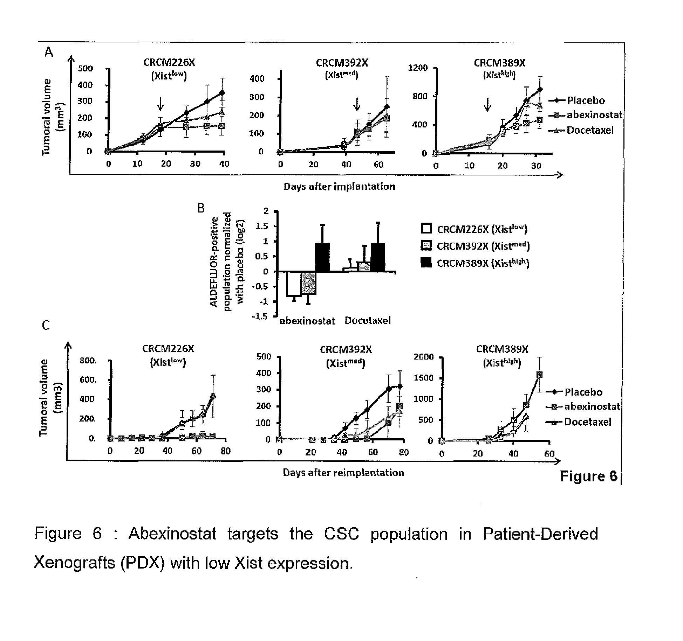

Tumor growth was compared with that of placebo-treated controls. An inhibition of the CRCM226X (Xist^{low}) PDX growth after abexinostat and docetaxel treatment was observed. Docetaxel and abexinostat treatment had no or discrete effect on the CRCM392X (Xist^{med}) and CRCM389X (Xist^{high}) PDX growth (Figure 6A). After three weeks of treatment, the animals were sacrificed and the proportion of ALDEFLUOR-positive CSCs was measured in each residual tumor (Figure 6B). All PDX models presented an increase in the ALDEFLUOR-positive population isolated from docetaxel-treated tumors compared to the untreated control. In contrast, only PDXs with a low or medium Xist expression treated with abexinostat presented a two-fold decrease of the ALDEFLUOR-positive population whereas abexinostat treatment induced an increase of the ALDEFLUOR-positive population of CRCM389X (Xist^{high}). To functionally prove the reduction of the CSC population in the abexinostat-treated tumors with low Xist expression, the ability of treated cells to form tumors *in vivo* by reimplantating cells from treated PDXs into secondary mice was determined. Tumorigenicity is directly related to the presence of CSCs and this assay gives an estimate of the proportion of residual tumorigenic CSCs. For each treatment condition (placebo, abexinostat, docetaxel), 1,000 cells isolated from treated-tumors were reimplanted. Cells isolated from abexinostat-treated PDXs showed an incapacity to regenerate a tumor for CRCM226X (Xist^{low}) and a delay in tumor regrowth for CRCM392X (Xist^{med}) compared to the cells isolated from placebo-treated tumors (Figure 6C). In sharp contrast, cells isolated from docetaxel-treated tumors showed a tumor regrowth comparable to placebo-treated tumors. Interestingly, for CRCM389X (Xist^{high}), cells isolated from abexinostat-treated tumors presented a higher regrowth kinetic compared to cells isolated from docetaxel- and placebo-treated tumors (Figure 6C). These results confirm the ability of the abexinostat treatment to target the CSC population *in vivo* and confirm that X chromosome numbers may influence drug-response with a better CSC eradication in PDX model with X chromosome monosomy compared to PDX model with X chromosome normosomy.

## Claims

1. A method of evaluating chemotherapy sensitivity in a patient in need thereof, comprising :
(a) assessing the number of X chromosome in a sample from a patient ; and
(b) comparing the number of X chromosome in (a) with the number of X chromosome reference from a control sample,
wherein a number of X chromosome that is identical or inferior to the reference is indicative of chemotherapeutic sensitivity in the patient.

2. A method of evaluating chemotherapy sensitivity in a patient in need thereof, comprising :
(a) assessing the level of expression of XIST gene in a sample from a patient ; and
(b) comparing the level of expression of XIST gene in (a) with the level of expression of reference XIST gene from a control sample,
wherein a level of expression of XIST gene that is inferior to the reference is indicative of a chemotherapeutic sensitivity in the patient.

3. Method of claim 1, wherein the number of X chromosome reference is the X chromosome monosomy.

4. Method of claim 1, wherein the number of X chromosome reference is the X chromosome disomy.

5. Method of claim 2, wherein the level expression of reference XIST gene is the level of expression of XIST gene in a disomic sample.

6. The method of claim 1, wherein the number of X chromosome that is higher than the number of X chromosome reference is indicative of a patient's potential resistance to chemotherapy.

7. The method of claim 2, wherein the level of expression of XIST gene that is identical or higher than the level of expression of reference XIST gene is indicative of a patient's potential resistance to chemotherapy.

8. The method of any one of claim 1 or 2, wherein the patient has breast cancer, ovarian cancer, cervical cancer, lymphoma and hematology diseases.

9. The method of claim 8, wherein the patient has cancer related to cancer stem cells.

10. The method of claim 9, wherein the patient has cancer related to breast cancer stem cells.

11. The method of any one of claim 1 or 2, wherein the chemotherapy sensitivity is sensitivity to treatment with a HDAC inhibitor.

12. The method of claim 11, wherein the HDAC inhibitor is selected from the group consisting of : abexinostat, vorinostat, belinostat, panobinostat, entinostat, mocetinostat, resminostat, givinostat, quisinostat, romidepsin, valproic acid.

13. The method of any one of claim 1 or 2, wherein the level of expression of XIST gene or the number of X chromosome is measured in a tumor sample from the patient.

14. The method of claim 13, wherein the patient tumor sample is a breast tumor sample.

15. The method of claim 2, wherein the level of expression of XIST gene is measured by determination of the level of transcription using DNA array, quantitative RT-PCR or RNA-FISH.

16. The method of claim 1, wherein the number of X chromosome is measured by SKY, M-FISH, array CGH or FISH on paraffin section.

17. A kit comprising at least one pair of primers specific for XIST gene that is a marker for chemosensitivity in a patient that has a cancer, at least one reagent for amplification of the XIST gene and instructions for use.

18. A kit comprising at least one probe specific for the number of X chromosome or specific for XIST gene that both are markers for chemosensitivity in a patient that has a cancer and instructions for use.

19. The kit of any one of claim 17 or 18, wherein the marker for chemosensitivity is a marker for sensitivity to a HDAC inhibitor.

20. The kit of any one of claim 17 or 19, wherein the kit is a quantitative RT-PCR kit.

21. The kit of any one of claim 18 or 19, wherein the kit is a FISH on paraffin section kit or RNA-FISH kit.

22. Use of XIST or number of X chromosome for assessing a patient sample in vitro for susceptibility to treatment with an HDAC inhibitor.

23. An HDAC inhibitor for use in treating a cancer in a patient, wherein the patient is identified with the method of any one of claim 1 or 2.

24. Abexinostat for use in treating a breast cancer related to cancer stem cells, wherein the patient is identified with the method of any one of claim 1 or 2.
